Europäisches Patentamt

European Patent Office

Office européen des brevets

(19)

(11) Publication number: **0 258 901**
**A2**

(12) **EUROPEAN PATENT APPLICATION**

Application number: 87112907.8

(51) Int. Cl.4: **A61B 17/32**

(22) Date of filing: **03.09.87**

(30) Priority: **03.09.86 JP 205800/86**

(43) Date of publication of application:
**09.03.88 Bulletin 88/10**

(84) Designated Contracting States:
**DE FR GB IT**

(71) Applicant: **TONOKURA IKA KOGYO CO. LTD**
**3-1, 5-chome Hongo**
**Bunkyo-ku Tokyo(JP)**

(72) Inventor: **Kobayashi, Hitoshi**
**36-26, 3-chome Sekizawa**
**Fujimi-shi Saitama-ken(JP)**
Inventor: **Kurihara, Mamoru**
**8-17, 4-chome Nishikoiwa**
**Edogawa-ku Tokyo(JP)**
Inventor: **Machida, Haruhiko**
**10-7, 4-chome Nakaochiai**
**Shinjuku-ku Tokyo(JP)**

(74) Representative: **LOUIS, PÖHLAU, LOHRENTZ &**
**SEGETH**
**Ferdinand-Maria-Strasse 12**
**D-8130 Starnberg(DE)**

(54) **Water jet operating apparatus and liquid supply unit line used for the apparatus.**

(57) An injection hole and a suction hole are formed in a handpiece, and a liquid supply source is connected to the injection hole via a supply line. The supply line includes an elastically deformable tube disposed at the midway. Liquid within the liquid supply source is supplied by pressure to the injection hole by means of an external pump such as a roller pump acting to the pump tube from the outside.

*FIG. 1*

EP 0 258 901 A2

## WATER JET OPERATING APPARATUS AND LIQUID SUPPLY UNIT LINE USED FOR THE APPARATUS

Background of the Invention

1. Field of the Invention

The present invention relates to a medical apparatus for surgical operation and, more particularly, to a water jet operating apparatus for cutting or cutting off organs etc., or washing internal cavities and outer surfaces of the human body by injection liquid as well as drawing out by suction separated tissues or the like produced thereby. The present invention also relates to a liquid supply unit line used for such a water jet operating apparatus.

2. Description of the Prior Art

There have been effectively used as a conventional operating means for cutting or cutting off organs or the like, a steel knife, an electrical knife, an ultrasonic knife, and a laser knife according to the purpose.

The steel knife is widely used for many surgical operations because of low costs and easy handling, but it has drawbacks that all tissues are cut without distinction. Accordingly, there is a risk that blood vessels, nerves or the like may be cut inadvertently, and the handling requires prudence and skill.

The electric knife can be handled easily and has been spread to some extent, but there is a risk that it may damage the affected part by the heat or produce a burn upon handling it.

The ultrasonic knife has the merit that the affected tissues can be cut without cutting blood vessels, nerves, etc. However, its cost is expensive and the handling is complicated. And the apparatus itself is likely to cause troubles.

The laser knife is excellent in that surgical opration can be performed without contacting with the affected part, an injury inflicted to the affected part is small, and it produce the effect of arrest of bleeding. However, the apparatus itself is at a high price. Besides, it may offer a difficulty to control over a depth of cutting so that it might incur the risk of massive bleeding on account of indiscriminate cutting of tissues.

As mentioned above, the operating apparatuses have the merits and the demerits. Accordingly, scrupulous care is required upon handling such apparatuses.

Recently, various investigations have been made to improve the above mentioned operating apparatuses. For exemple, a method for applying a pressurized liquid jet flow to some restricted parts has been investigated and applied clinically (British Journal of Surgery, Vol.69, pages 93-94).

With the method using such pressurized liquid jet flow, it is possible to separate the affected part from surrouding tissues without cutting blood vessels or cords of nerves by adjusting the liquid pressure and the injection amount to a suitable range. It is also possible to withdraw the separated tissues together with the injected liquid around the affected part.

Accordingly, the method may be applicable to a wide variety of fields and may separate the objective tissues from the organs using pressurized liquid both at high pressures, for example, of about 10 MPa, and at low pressures, for example, of not more than 1 MPa. Using such low-pressure liquid, this method is applicable to a separation of parenchyma from cords at a soft part and to washing and so on. The liquid used in the case may be preferably psychological saline or any other one exerting no adverse effect on the human body. Besides, cooling or heating can be conducted at the same time as the operation of the apparatus by adjusting the temperature of the liquid properly. Studies and experiments on this method have now been conducted to develop wide usages with various advantages.

However, the above mentioned method has the following disadvantages. Namely, when the pressurized liquid is injected and striken against the affected part, tissues separated from the part arecaused to be admixed with water and air are and the resulting mixture may produce foams by a strong stirring action caused by the pressurized liquid jet. As a result, it becomes difficult to precisely judge the affected part to be operated, and the scattering of foams pollutes the surroundings and causes the visibility to become poor and be lost.

Most recently, a water jet operating apparatus in which a suction is conducted at the same time as the liquid injection has been proposed to solve the problems encountered with the conventional surgical apparatus.

However, such an apparatus has drawbacks that its structure becomes complicated as a whole and the handling or operation is troublesome because sterilization of the apparatus is required prior to each surgical operation especially where operation must be performed under aseptic conditions. In addition, there is also a problem that a , consumption amount of the liquid becomes large.

A conventional water jet operating apparatus uses a handpiece having an injection hole and a suction hole opened at the front side thereof. To the injection hole is connected a supply line extending from a liquid supply source, and to the suction hole is connected a suction means such as a vacuum pump via a suction line.

A plunger pump is connected at the midway of the supply line. Namely, a pump chamber whose volume is varied with the movement of a reciprocating plunger forms a part of the supply line. The pressurized liquid generated by the pump is supplied to an accumulator to absorb the pulsating components and thereafter is supplied to the injection hole. The handpiece further includes a change-over valve for connecting or disconnecting the supply line to the injection hole. When the injection flow is not needed, the pressurized liquid generated by the plunger pump is directly exhausted from the supply line to the outside. The exhaust of the pressurized liquid is conducted for the protection of the supply line because the plunger pump used is of a high pressure type. In more detail, the use of the plunger pump is necessarily accompanied with the use of a suction valve and an exhaust valve as a check valve. As a result, the pressurized liquid at high pressure generated by the plunger pump is continuously supplied to the downstream side of the exhaust valve. For this reason, to protect the supply line of the downstream side of the exhaust valve, the pressurized liquid must be exhausted to the outside.

With the above conventional water jet operating apparatus, the plunger pump and the accumulator must be disassembled for sterilization upon the re-use. The sterilization work and the set-up of the apparatus for the re-use take much time and labor. In addition, as the pressurized liquid is exhausted to the outside when injection flow is not needed, the consumption amount of liquid becomes large. Besides, as the plunger pump is necessarily accompanied with the suction valve and the exhaust valve, and the accumulator is arranged in the apparatus, the apparatus become rather complicated in structure.

Summary of the Invention

Accordingly, it is a principle object of the present invention to provide a water jet operating apparatus which can be readily sterilized and set up.

It is further object of the present invention to provide a water jet operating apparatus which can reduce a consumption amount of liquid and simplify the construction as a whole.

It is still further object of the present invention to provide a unit line for supplying liquid used suitably for the above water jet opetating apparatus, especially suitable as a disposable type one.

To attain the above-mentioned objects, the water jet operating apparatus of the present invention essentially comprises a handpiece having an injection hole and a suction hole both opened at the front end thereof, a liquid supply source for storing liquid to be injected from the injection hole of the handpiece, a supply line connecting the injection hole of the handpiece to the liquid supply source, and having an elastically deformable pump tube disposed at the midway, and the portion thereof between the pump tube and the handpiece being formed as a pressure tube, an external pump for supplying by pressure the liquid within the liquid supply source toward the handpiece by acting to the pump tube from the outside, and a suction means connected to the suction hole of the handpiece via a suction line.

In the present invention, an injection flow of pressurized liquid is generated in a manner that the liquid is not brought into direct contact with the pump by allowing the pump tube itself to constitute a pump chamber. As a result, the pump is no longer required to disassemble for sterilization, and set-up of the apparatus can be readily done.

It is preferable to use a roller pump as the external pump. In this case, the injection flow becomes a pulsating one. However, the injection flow need not be necessarily a steady one for cutting or washing organs or the like so that an accumulator can be omitted.

When the liquid is not injected from the injection hole, it can be prevented to exert a large pressure to the pump tube and the pressure tube at the downstream relative to the pump tube, owing to the back-flow in the pump tube.

The pressure of the injection flow used in the present invention may be preferably set at a value within the range between 2-30 $kgf/cm^2$. For washing, the pressure is set at as low as about 2 $kgf/cm^2$. For cutting organs of the human body, the pressure of at most 30 $kgf/cm^2$ is sufficient and such pressure is also sufficient for the protection of the liquid supply line. The pressure adjustment within the above pressure range may be done, for example, by adjusting a rotational speed of the external pump.

It is also preferable to dispose a return line in parallel with the supply line for the protection of the supply line. When the liquid is not injected from the injection hole, it is advantageous that the pressurized liquid from the supply line is returned to the liquid supply source via the return line.

A unit line for the water jet operating apparatus, of the present invention comprises a handpiece and a supply line for supplying liquid to the handpiece. Namely, the unit line of the present invention essentially comprises a handpiece having an injection hole and a suction hole both opened at the front end thereof, a liquid supply tube whose one end being connected to the injection hole of the handpiece, including an elastically deformable pump tube disposed at the midway, and the portion thereof between the pump tube and the handpiece is formed as a pressure tube, a needle connected to the other end of the liquid supply tube, and an air trap connected to the liquid supply tube between the needle and the pump tube.

The unit line becomes low in cost enough as a disposable one. Accordingly, upon successive operations, if such unit lines are prepared in several sets in advance, one operation can be done after another very easily in a short time. Namely, the set-up of the unit line can be completed without special sterilizing work, only by setting the pump tube to the external pump and connecting the suction hole to the suction means.

Additional objects and features of the present invention will be apparent from the following descriptions of the preferred embodiments.

Brief Description of the Drawings

FIG. 1 is an entire systematic view of an embodiment according to the present invention;

FIG. 2 is a perspective view illustrating a practical form of the embodiment of FIG. 1;

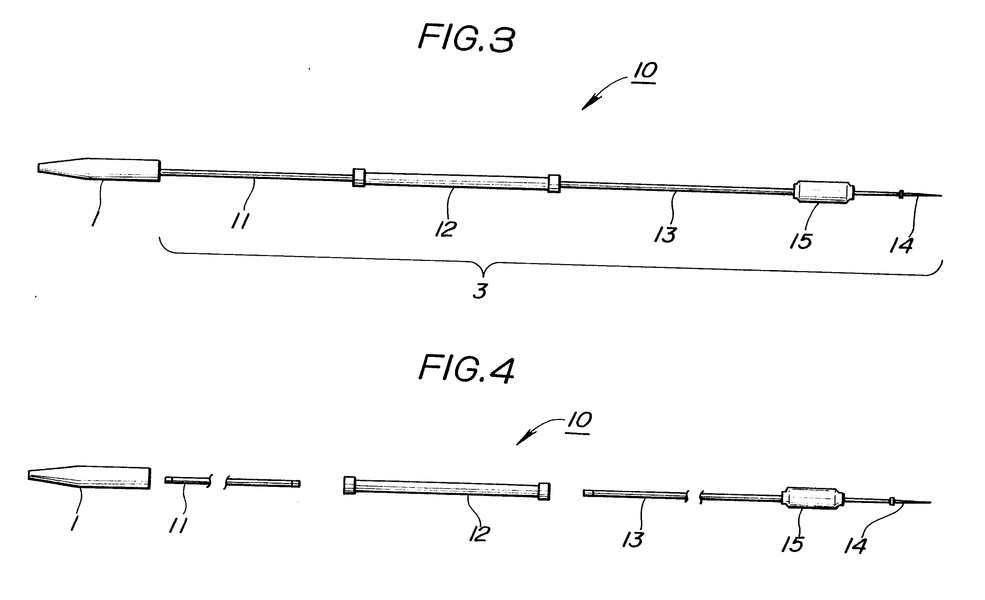

FIG. 3 is a side view of a unit line;

FIG. 4 is an exploded view of the unit line of FIG. 3;

FIG. 5 is a side view in cross section illustrating an example of a handpiece;

FIG. 6 is a view of the handpiece in the direction of the rightward arrow in FIG. 5;

FIG. 7 is a side view in cross section of a pump tube and a pressure tube;

FIG. 8 is an entire systematic view of another embodiment of the present invention;

FIG. 9 is a side view in parallel cross section of the handpiece of FIG. 8; and

FIG. 10 is a sectional view of the principle part of FIG. 9.

Detailed Description of Preferred Embodiments

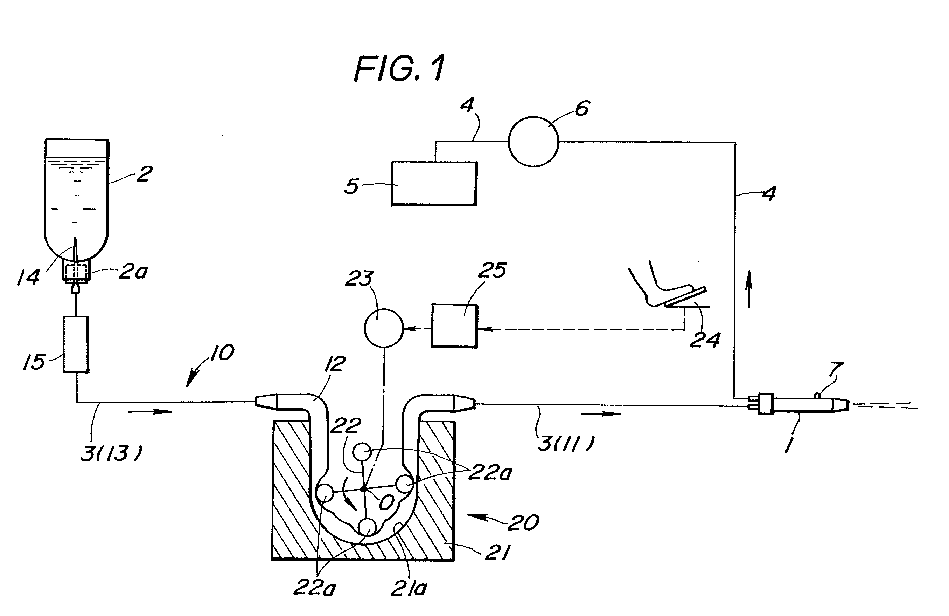

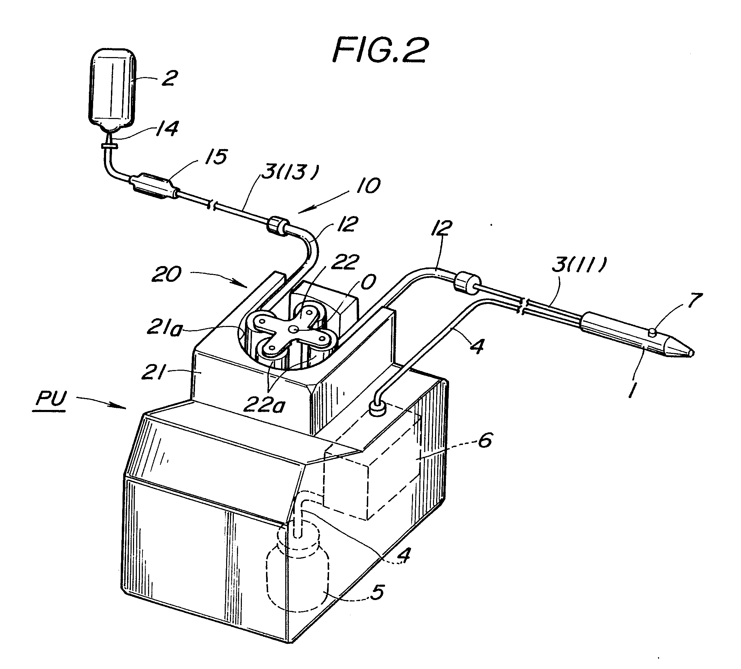

In FIGS. 1 and 2, reference numerals 1 and 2 represent a handpiece and a reservoir as a liquid supply source, respectively. The handpiece 1 has an injection hole and a suction hole both of which are opened at the front end thereof, as explained hereinafter. The handpiece 1 and the reservoir 2 are connected together via a supply line 3 for supplying liquid. The handpiece 1 is also connected to a dust box 5 via a suction line 4. On the midway of the suction line 4, a suction pump 6 is arranged as a suction means. The supply line 3 supplies liquid to the injection hole of the handpiece 1, and the connection and disconnection between the injection hole of the handpiece 1 and the supply line 3 can be changed over by operating a knob of a change-over valve 7 disposed in the handpiece 1. The suction line 4 always communicates with the suction hole of the handpiece 1.

Together with the handpiece 1, the supply line 3 forms a unit line 10. The supply line 3 comprises a first tube 11, a second tube 12 and a third tube 13, in turn, starting from the handpiece 1 side. A needle 14 is connected to the upstream end of the third tube 13, and air trap 15 is arranged on the midway of the same. The needle 14 which forms the upstream end of the supply line 3 penetrates through a stopper 2a made of flexible material which corks the reservoir 2, and extends to the inside of the reservoir 2. The air trap 15 has a relatively large capacity to store air therein, and is made from transparent plastics to allow an operator to observe a flow state etc. of the liquid from outside. Optionally, a filter having a mesh size of order of hundred micron meters may be disposed within the air trap 15.

The second tube 12 forms a pump tube and is made from flexible and deformable material enough to withstand against pressure or mechanical external force. The first tube 11 is made from flexible material to withstand against pressure and not to disturb the movement of the handpiece 1. Also, from a view point of not disturbing the movement of the handpiece 1, at least the portion near by the handpiece 1, of the supply line 4 is formed by a flexible tube.

The second tube 12 serving as the pump tube is set to a roller pump 20 as an exterior pump. The roller pump 20 comprises a main body 21 having a recessed portion 21a formed in substantially semi-circular shape, and a rotary member 22 disposed within the recessed portion 21a and provided with plural rollers 22a arranged circumferentially at regular intervals. The rotational center 0 of the rotary member 22 coincides with the center of the recessed portion 21a of substantially semi-circular shape, and the closest distance between the inner wall of the recessed portion 21a and the roller 22a is set at a relatively smaller value than the outer diameter of the second tube 12 in free state. The rotary member 22 is rotated by an electric motor 23. The rotational speed of the rotary member 23 is adjusted by a foot-pedal switch 24 operated by the foot of a doctor performing a surgical operation. Namely, the operating amount of the foot-pedal switch 24 is fed to a motor control circuit 25, whereby the motor control circuit 25 controls the electiric motor 23 so that the rotational speed reaches the value corresponding to the operating amount of the foot-pedal switch 24.

Respective elements 5, 6, 20-23 and 25, mentioned above, having a connection with the pump are combined into one unit as a pump unit PU, as shown in FIG. 2.

The operation of the water jet operating apparatus having the above construction is as follows. The second tube 12 of the supply line 3 namely the pump tube is arranged along the inner wall of the recessed portion 21a of the roller pump 20, as shown in FIGS. 1 and 2. With such arrangement, in use, the rotary member 22 is rotated in counter-clockwise direction in FIG. 1. By this rotation, the second tube 12 is compressed by the rollers 22a so that its diameter is reduced, and the compressed portion is successively moved to the handpiece 1 side. The portion whose diameter has been once reduced by compression can be recovered to the original state when the roller 22a moves away from it. Consequently, liquid within the reservoir 2 is drawn out into the second tube 12 and fed to the handpiece 1.

By depression of the knob of the change-over valve 7 by a finger, the liquid fed by pressure to the handpiece 1 can be in communication with the injection hole, so that the liquid is injected as an injection flow from the injection hole. Then, the puressure of the injection flow is adjusted within a range between 2 and 30 kgf/cm$^2$ by operating the foot-pedal switch 24. Where the pressure value of the injection flow is small, for example, 2kgf/cm$^2$, this apparatus can be used for washing, and where the same is large, for example, within the range between 20-30 kgf/cm$^2$, it can be used for cutting organs. On the other hand, the front end of the handpiece 1 is brought close to organs, and liquid or separated pieces such as amputated pieces around the organs can be drawn out from the suction hole and discharged to the dust box 5.

When the depressed knob of the change-over valve 7 is released, the liquid injection from the handpiece 1 is stopped. At the same time, the depression of the foot-pedal switch 24 is generally released. In this case, the pressure generated within the second tube 12 becomes small. Accordingly, there is no problem about the protection (durability) of the supply line 3 (especially the first and the second tubes 11, 12). On the other hand, the foot-pedal switch 24 is strongly depressed at the state where the liquid injection is stopped, then the pressure generated within the second tube 12 becomes rather large. However, even if such large pressure generates, it is escaped from the second tube 12 side to the third tube 13 side. Accordingly, there is also no problem about the protection for the first and the second tubes 11, 12.

The unit line 10 will then be described more in detail.

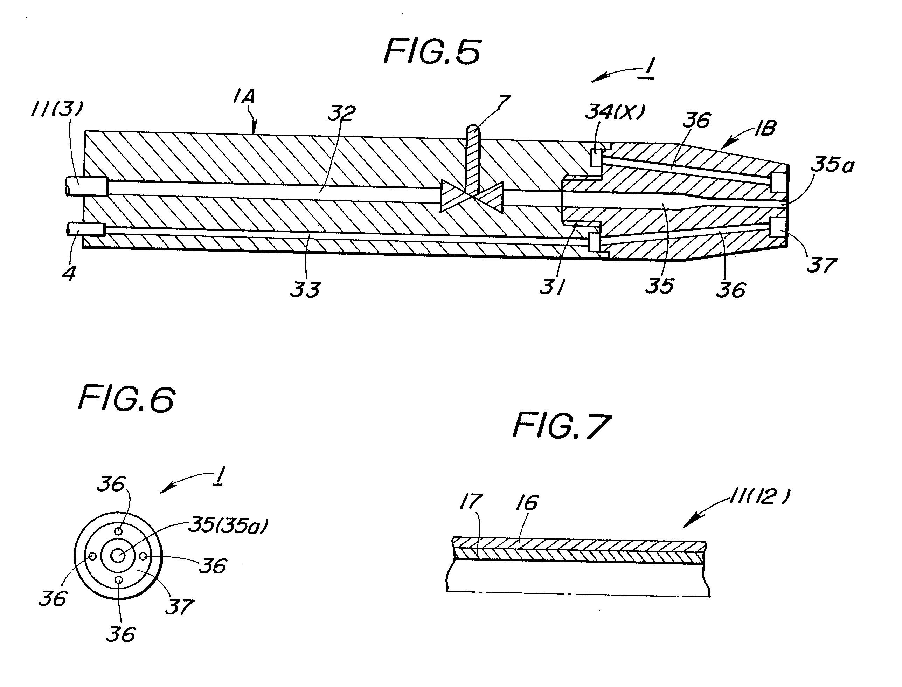

As shown in FIG. 4, the unit line 10 essentially comprises four separate pieces 1, 11, 12 and 13, and the connection between each pieces can be done, for example, by using lure-male and female or by usual threaded insert method. Each pieces 1, 11, 12 and 13 are connected together upon setting up. The needle 14 and the air trap 15 have been connected in advance to the third tube 13. Alternatively, it may be used a unit in which respective elements 12, 13, 14 and 15 have been connected together in advance as one body.

Preferably, the first tube 11 as a pressure tube and the second tube 12 as a pump tube, especially the latter one to which is applied an exterior force by the roller pump 20 may be of double structure comprising an outer tube 16 and an inner tube 17, as shown in FIG. 7. As the outer tube 16, there may be used, for example, a polyethylene tube or a vinylchloride tube, each including nylon braid (reinforced by retiform nylon fibers). As the inner tube 17, there may be used , for example, a polyethylene tube or vinylchloride tube (nylon braid may not be necessarily included). Of course, the first and the second tubes 11, 12 are not restricted to the double structure, and more higher multi-tube structure or single structure may be used therefor. The third tube 13 may be made from material such as vinylchoride, polyethylene, nylon and the like.

The handpiece 1 is formed in a shape of a pencil slightly thick, as a whole, to be handled easily with a single hand, as shown in FIGS. 5 and 6. Except the change-over valve 7, the handpiece 1 comprises two separate pieces, that is, a body portion 1A and a tip portion 1B, both of which may be made from synthetic resin such as Delrin. The tip portion 1B is screwed into the body portion 1A. The screw-connected portion is indicated by reference numeral 31 in the figure.

The body portion 1A has a supply path 32 and a suction path 33 formed therein, both of which are opened at the front and rear ends of the body portion 1A. At the front end of the body portion 1A, an annular groove 34 is formed to surround the supply path 32. Where the tip portion 1B is connected to the body portion 1A, the annular groove 34 forms a closed space X isolated from outside. The suction path 33 is opend to the annnular groove 34 (closed space X).

On the other hand, the tip portion 1B has an injection hole 35 opened at the the front end thereof and a plurality (four in this embodiment) of suction holes 36. On the front end of the tip portion 1B, an annular groove 37, to which the suction holes 36 are opened, is formed to surround the injection hole 35. Namely, the annular groove 37 substantially forms the suction hole opened at the end of the handpiece 1. Where the tip portion 1B is connected to the body portion 1A, the injection hole 35 communicates with the supply path 32 and the suction hole 36 communicate with the annular groove 34 (closed space X). The front portion of the injection hole 35 is formed as a nozzle 35a having a reduced diameter which is set at about 0.1-0.5 mm, taking account of injection amount and pressure.

The change-over valve 7 opens and shuts off the supply path 32. A sprue type valve may be used at the change-over valve 7. The first tube 11 is connected to the rear end of the supply path 32, and the suction line 4 is connected to the suction path 33.

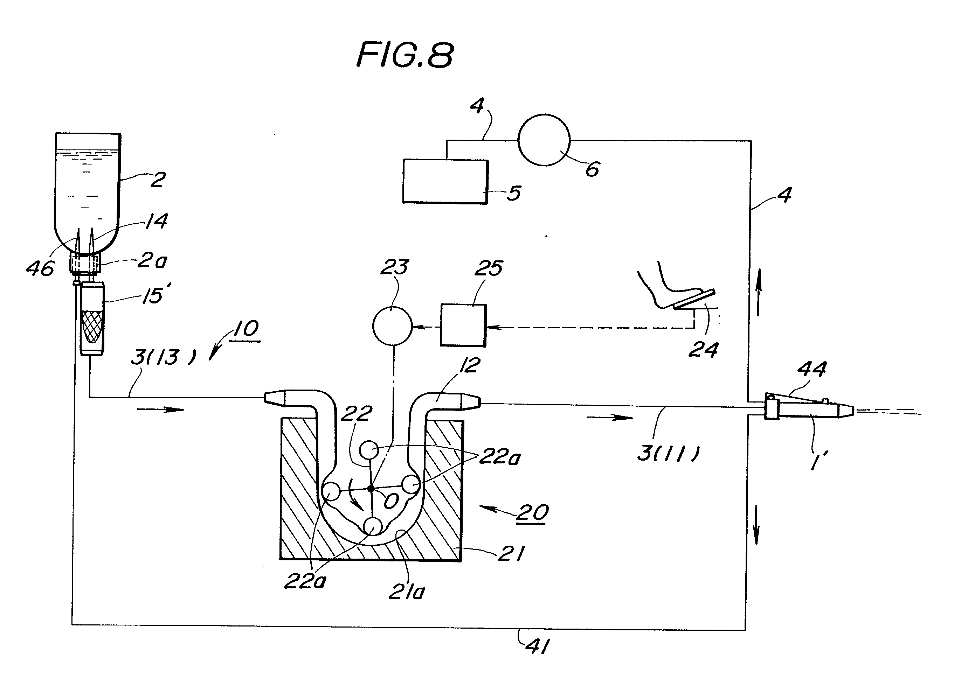

FIG. 8 shows another embodiment according to the present invention, in which the same reference numerals denote the same elements in the above-mentioned embodiment and the explanation for them is omitted herein.

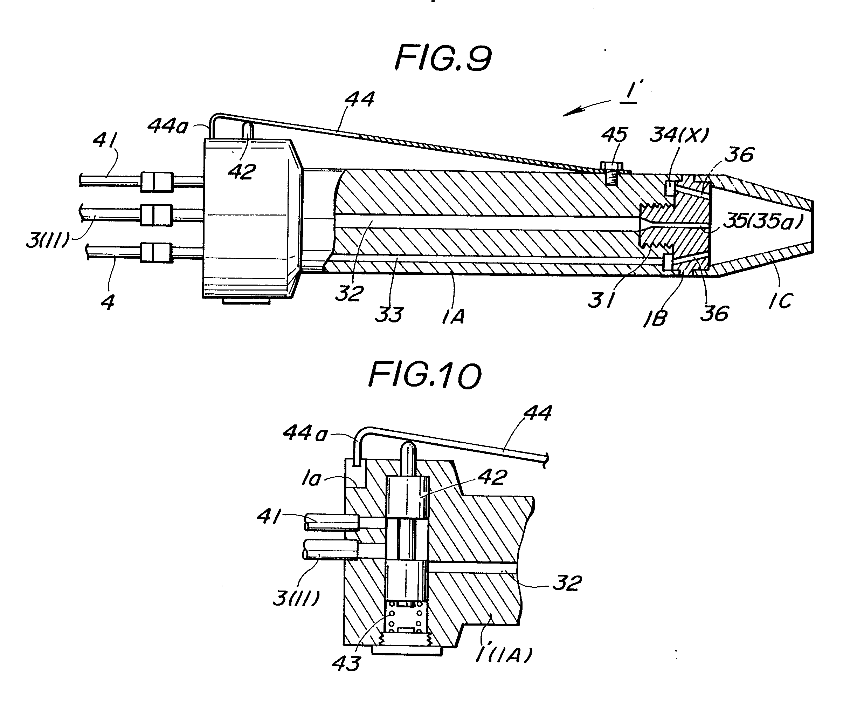

In this embodiment, a liquid return line 41 is arranged in parallel with the liquid supply line 3. When liquid injection from the handpiece 1' is not performed, puressurized lipuid from the supply line 3 returns via the return line 41 to the reservoir 2. To this end, the handpiece 1' is provided with a change-over valve 42, as will be described later, explained later by which the supply line 3 is selectively communicated with the injection hole 35 or the return line 41. The change-over valve 42, which may be sprue type one as shown in FIG. 10, is usually urged upwardly by a spring 43, so as to communicate the supply line 3 with the return line 41. When the change-over valve 42 is depressed and moved downwardly, the supply line 3 communicates with the injection hole 35. The return line 41 may be formed by a flexible tube made from plastics and the like, and its one end opposite to the handpiece 1' is connected to a needle 46.

The handpiece 1' includes a tubular guide cover 1C detachably fitted on the outer periphery of the tip portion 1B, in addition to two separate members mentioned in the former embodiment, namely the body portion 1A and the tip portion 1B. More specifically, with this embodiment, the length of the chip portion 1B can be reduced, as compared with that of the former embodiment and the annular groove 37 can be omitted. Both the injection hole 35 and the suction hole 36 are opened to within the guide cover 1C the inner diameter portion of which is tapered and gradually reduced toward its front end. The advantages of this embodiment are that the length of the tip portion 1B provided with the injection hole 35 and the suction hole 36 can be reduced considerably and the construction of the same can be simplified. Accordingly, it is advantageous in manufacturing.

For the operation of the change-over valve 42, the handpiece 1' includes a spring plate 44. The spring plate 44 is of a rectilinear shape extending along the longitudinal direction of the handpiece 1', and its front end portion is fixed by a screw 45 to the outer periphery of the front end portion of the body portion 1A. The rear end of the spring plate 44 is made free and can access to and go away from the outer periphery of the body portion 1A. Usually, the rear end of the spring plate 44 is remote from the outer periphery of the body portion 1A due to its elastic recovery force, as shown in FIGS. 9 and 10. When the spring plate 44 is depressed, namely elastically displaced to approach to the outer periphery of the body portion 1A, the change-over valve 42 moves downwardly by the spring plate 44. The rear end portion of the spring plate 44 is bent by about 90 degrees to form a bent portion 44a. By abutting the bent portion 44a against the shoulder (stopper) 1a in a notch formed in the body portion 1A, further displacement can be restricted. The advantage of provision of such spring plate 44 is that the change-over valve 42 can be moved downwardly by depressing at any points over the most length of the spring plate 44.

Having described the present invention in connection with embodiments thereof heretofore, there can be used at the external pump any suitable pumps other than the roller pump, for example, a finger type one.

It is to be understood that the present invention is not limited to the above embodiments and many changes and modifications can be made within the scope and sprit in the appended claims.

**Claims**

(1) A water jet operating apparatus comprising; a handpiece having an injection hole and a suction hole both opened at the fornt end thereof; a liquid supply source for storing liquid to be injected from the injection hole of the handpiece; a supply line connecting the injection hole of the handpiece to the liquid supply source, and having an elastically deformable pump tube disposed at the midway, and the portion thereof between the pump tube and the handpiece being formed as a pressure tube;

an external pump for supplying by pressure the liquid within the liquid supply source toward the handpiece by acting to the pump tube from the outside; and

a suction means connected to the suction hole of the handpiece via a suction line.

(2) The water jet operating apparatus according to claim 1, further comprising a return line connecting the liquid supply source to the handpiece, in parallel with the supply line, and the handpiece further including a change-over valve for communicating the supply line with the injection hole or the return line selectively,

(3) The water jet operating apparatus according to claim 1, in which the external pump is a roller pump.

(4) The water jet operating apparatus according to claim 1, in which the pressure of the injection liquid from the injection hole is set at a value between 2 and 30 kgf/cm².

(5) The water jet operating apparatus according to claim 4, in which the pressure of the injection liquid from the injection hole is adjusted by adjusting a rotational speed of the external pump.

(6) The water jet operating apparatus according to claim 5, in which the rotational speed of the external pump is adjusted by a foot-pedal switch.

(7) The water jet operating apparatus according to claim 1, in which the supply line includes an air trap disposed at the upstream side relative to the pump tube.

(8) The water jet operating apparatus according to claim 1, in which the liquid supply source is a bottle storing liquid and corked by a stopper made from a flexible member and the upstream end of the supply line is formed by a needle which penetrates through the stopper.

(9) A unit line for supplying liquid used for a water jet operating apparatus comprising:

a handpiece having an injection hole and a suction hole both opened at the front end thereof;

a liquid supply tube whose one end being connected to the injection hole of the handpiece, including an elastically deformable pump tube disposed at the midway, and the portion thereof between the pump tube and the handpiece is formed as a pressure tube;

a needle connected to the other end of the liquid supply tube; and

an air trap connected to the liquid supply tube between the needle and the pump tube.

(10) The unit line according to claim 9, in which the handpiece includes a change-over valve for changing over the connection and the disconnection between the liquid supply tube and the injection hole,

(11) The unit line according to claim 9, further comprising a return tube whose one end being connected to the handpiece and whose other end being connected to a needle, and the handpiece including a change-over valve for communicating the liquid supply tube with the injection hole or the return tube selectively,

(12) The unit line according to claim 9, in which the pump tube has double structure comprising an outer tube and an inner tube.

(13) The unit line according to claim 9, in which the pressure tube has double structure comprising an outer tube and an inner tube.

(14) The unit line according to claim 12, in which the outer tube is formed by polyethylene or vinylchloride tube reinforced by nylon braid.

(15) The unit line according to claim 12, in which the inner tube is formed by polyethylene or vinylchloride tube.

(16) The unit line according to claim 13, in which the outer tube is formed by polyethylene or vinylchloride tube reinforced by nylon braid.

(17) The unit line according to claim 13, in which the inner tube is formed by polyethylene or vinylchloride tube.

(18) The unit line according to claim 9, in which the liquid supply line is comprised by connecting together at least three tubes comprising a first tube forming the pressure tube, a second tube forming the pump tube and a third tube forming an upstream tube between the pump tube and the needle.

(19) The line unit according to claim 9, in which the handpiece is made from synthetic resin and formed in shape of a pencil as a whole.

(20) The line unit according to claim 11, in which the change-over valve is disposed at the rear end portion of the handpiece, the handpiece further includes a spring plate for operating the change-over valve, extending along the longitudinal direction thereof, and the spring plate is so designed that its one end is fixed to the outer periphery at the front end portion of the handpiece and the other end is free, whereby when the spring plate is elastically displaced in the direction approaching to the outer periphery of the handpiece, the change-over valve communicates the liquid supply tube with the injection tube.

FIG.1

0 258 901

# FIG.2

0 258 901

FIG.3

10

11 12 13 15 14

3

FIG.4

10

11 12 13 15 14

# FIG.5

# FIG.6

# FIG.7

# FIG.8

0 258 901

# FIG.9

44a · 44 · 45 · 34(X) · 36 · 35(35a) · 41 · 42 · 3(11) · 4 · 32 · 33 · 1A · 31 · 1B · 36 · 1C

# FIG.10

44a · 44 · 1a · 42 · 41 · 3(11) · 32 · 43 · 1'(1A)

0 258 901